# EUROPÄISCHE PATENTSCHRIFT

(11) **EP 1 485 120 B1**
(45) Veröffentlichungstag und Bekanntmachung des Hinweises auf die Patenterteilung: **11.01.2006**
(21) Anmeldenummer: 03708173.4
(22) Anmeldetag: 04.03.2003
(51) Int. Cl.: A61K 38/42, A61P 17/02

(54) **VERWENDUNG EINES ODER MEHRERER VON PLASMA UND ZELLWANDBESTANDTEILEN BEFREITEN NATÜRLICHEN ODER MODIFIZIERTEN SAUERSTOFFBINDER ZUR EXTERNEN BEHANDLUNG OFFENER, INSBESONDERE CHRONISCHER WUNDEN**
USE OF ONE OR MORE NATURAL OR MODIFIED OXYGEN CARRIERS, DEVOID OF PLASMA AND CELLULAR MEMBRANE CONSTITUENTS, FOR EXTERNALLY TREATING OPEN, IN PARTICULAR CHRONIC WOUNDS
UTILISATION D'UN OU DE PLUSIEURS LIANTS D'OXYGENE NATURELS OU MODIFIES, EXEMPTS DE PLASMA ET DE CONSTITUANTS DE LA PAROI CELLULAIRE, POUR LE TRAITEMENT EXTERNE DE PLAIES OUVERTES, NOTAMMENT CHRONIQUES

(30) Priorität: 20.03.2002 DE 10212321
(43) Veröffentlichungstag der Anmeldung: 15.12.2004
(73) Patentinhaber: SanguiBioTech GmbH, 58455 Witten an der Ruhr (DE)
(72) Erfinder: BARNIKOL, Wolfgang, 55128 Mainz (DE); TESLENKO, Alexander, 58095 Hagen (DE)
(74) Vertreter: Müller, Claudia
(86) Internationale Anmeldenummer: PCT/EP2003/002193
(87) Internationale Veröffentlichungsnummer: WO 2003/077941

(56) Entgegenhaltungen:
- WO-A-02/00246
- WO-A-02/05754
- HINZ J ET AL: "Local treatment of non-healing and infected wounds with the biocatalytic activated oxygen carrier tetrachlorodecaoxide (TCDO)" MEDIZINISCHE WELT 1985 GERMANY, Bd. 36, Nr. 8, 1985, Seiten 210-215, XP001152691
- JONSSON K ET AL: "TISSUE OXYGENATION ANEMIA AND PERFUSION IN RELATION TO WOUND HEALING IN SURGICAL PATIENTS" ANNALS OF SURGERY, Bd. 214, Nr. 5, 1991, Seiten 605-613, XP009012115 ISSN: 0003-4932

## Beschreibung

### Gegenstand der Erfindung

Die vorliegende Erfindung betrifft die Verwendung eines oder mehrerer von Plasma und Zellwandbestandteilen befreiten natürlichen oder modifizierten Sauerstoffbinder, zur Herstellung eines Mittels, zur externen Behandlung von offenen, insbesondere chronischen Wunden. Als Sauerstoffbinder sind Hämoglobin oder Myoglobin humanen oder tierischen Ursprungs geeignet. Die Sauerstoffbinder können auch bevorzugt modifiziert sein. Geeignete Modifikationen sind Vernetzung, Umsetzung mit Polyalkylenoxiden, chemisch reaktiven oder chemisch nicht reaktiven Effektoren oder Kombinationen. Das Mittel kann auf den wunden Bereich insbesondere durch Sprühen einer wässrigen Lösung, enthaltend den oder die Sauerstoffbinder, aufgetragen werden Besonders wirksam können die Sauerstoffbinder bei chronischen Wunden infolge Gewebedegeneration, vor allem diabetischer Gewebedegeneration, eingesetzt werden.

### Hintergrund der Erfindung

Zur Behandlung von Wunden werden je nach Zustand unterschiedliche Methoden angewendet. Zunächst muss eine noch offene Wunde desinfiziert und somit gegen äußere negative Einflüsse geschützt werden. Dies kann mittels geeigneter Desinfektionslösungen oder Sprühverbänden oder auch durch Auftragen von Jodlösung erfolgen. Die eigentliche Wundheilung muss dann von innen erfolgen. Dies bedeutet, dass die noch vorhandenen Blutgefäße die zerstörten Gewebe ausreichend mit Substraten versorgen müssen, damit der gewebliche Reparaturmechanismus einsetzen kann.

Wunden können durch unterschiedlichste Faktoren verursacht sein, wie beispielsweise Verletzungen oder auch nach Operationen oder traumatischen Ereignissen.
Andererseits ist bekannt, dass Wundbildungen, insbesondere auch chronische Wunden, durch Erkrankungen hervorgerufen werden können, bei denen eine Degeneration und/oder Verengung großer und/oder kleiner Gefäße auftritt. Dies kann z.B. bei älteren Patienten durch langes Aufliegen (Dekubitus) erfolgen. Ein anderes Beispiel hierfür ist der Diabetes mellitus - die sogenannte Zuckerkrankheit - die eine nachweisbare Degeneration und Arteriosklerose (P. Carpenter, A. Franco, Atlas der Kapillaroskopie, 1983, Abbott, Max-Planck-Inst. 2, D-Wiesbaden) der großen und kleinen Gefäße (Makro- und Mikroangiopathie der Arterien) zur Folge hat. Hierbei konnte femer vor allem an der die Wunde umgebenden Haut durch Messung des sogenannten transkutanen Sauerstoffpartialdruckes eine Verkleinerung dieser Messgröße festgestellt werden. Das bedeutet, es liegt hier ein Sauerstoffmangel (Hypoxie), vor. Als kritischer Wert gilt 40mmHg (C.D. Müller et.al., Hartmann Wund Forum 1 ('99) 17-25).
Durch die Arterien fließt das Blut zu den Geweben, auch zur Haut. Es versorgt die Zellen ständig mit den lebensnotwendigen Substraten. Eine Degeneration dieser Gefäße führt zu einer Unterversorgung der Zellen mit Substraten und zu deren Absterben. Das letzte kleine scheinbar unbedeutende Wegstück von etwa 20 µm von den kleinsten Gefäßen (Kapillaren) zu den Zellen müssen die Substrate diffusiv oder filtrativ überwinden, eine Sonderrolle spielt hierbei der Sauerstoff, weil der Organismus im Umgang mit diesem Substrat besondere Probleme hat.

Es handelt sich hierbei um 3 Probleme:
(1) Sauerstoff ist zwar absolut lebensnotwendig (schon nach etwa 5 Minuten ist ein Mensch hirntot, wenn seinem Gehirn kein Sauerstoff zugeführt wird), aber der Sauerstoff ist zugleich hochtoxisch (ein mit reinem Sauerstoff beatmetes Neugeborene ist schon nach wenigen Tagen tot).
(2) Sauerstoff hat im wässrigen Milieu eine sehr kleine Löslichkeit. Dies führt gemäß dem 1. FICKschen Gesetz zu einer geringen Diffusivität des Sauerstoffs. Hinzu kommt noch eine Grundgesetzlichkeit der Diffusion, nämlich die Beziehung von SMOLUCHOWSKI und EINSTEIN, welche besagt, dass die Diffusionsgeschwindigkeit (des Sauerstoffs) mit steigendem Diffusionsabstand abnimmt. Die Diffusionskonstante des Sauerstoffs ist nun so klein, dass bereits bei einem Diffusionsabstand von nur 20 µm die Diffusionsgeschwindigkeit nur noch 5% des anfänglichen Wertes beträgt. Somit stellt eine Wasserschicht von nur 50 µm praktisch eine vollständige Sauerstoffisolierung für die Zellen dar. Sauerstoff ist gleichsam fußkrank. Die langen Wege im Organismus von der Lunge zur Zehenspitze wird er, gebunden am Hämoglobin, mit dem Blutstrom konvektiv transportiert und vermag nur so die großen Strecken in einer für den Organismus sinnvoll kurzen Zeit zu überwinden.
(3) Für Sauerstoff gibt es - im Gegensatz beispielsweise zur Glukose - keine Speicher im Körper; demnach muss dieses Substrat den Zellen jederzeit und schnell in ausreichender Menge zur Verfügung stehen; Sauerstoff ist ein lebensnotwendiges sogenanntes Sofortsubstrat.
Diese Probleme hat der Organismus mit Hilfe verschiedener Mechanismen gelöst. Die toxischen Wirkungen des Sauerstoffs werden dadurch vermieden, dass dieser sich an das Hämoglobin bindet und so harmlos bleibt. Zugleich wird der freie Sauerstoff verdünnt und verliert damit weiterhin sein schädliches oxidatives Potential. Trotzdem steht er in ausreichender Menge momentan zur Verfügung, weil die Bindung am Hämoglobin reversibel ist. Das Problem der geringen diffusiven Reichweite wird dadurch gelöst, dass der Organismus ein sehr feindisperses Gefäßnetz (Kapillarsystem) entwickelt hat, welches gewährleistet, dass im Mittel jede Zelle nur höchstens 25 µm von einer Kapillare entfernt ist; dadurch bleibt der Diffusionsweg des Sauerstoffs im Organismus unter der kritischen Länge von 50 µm. Stets kann zudem eine Zelle von mehreren Seiten diffusiv mit Sauerstoff versorgt werden; dies stellt einen Sicherheitsmechanismus dar. Die sofortige bedarfsgerechte - Sauerstoff darf nicht im Überschuss vorhanden sein, er wirkte sonst schädlich - Verfügbarkeit des Sauerstoffs wird im Organismus durch eine vaskuläre Regulation des Gefäßstromes erreicht, die die Durchblutung steuert und damit die Sauerstoffversorgung optimiert.

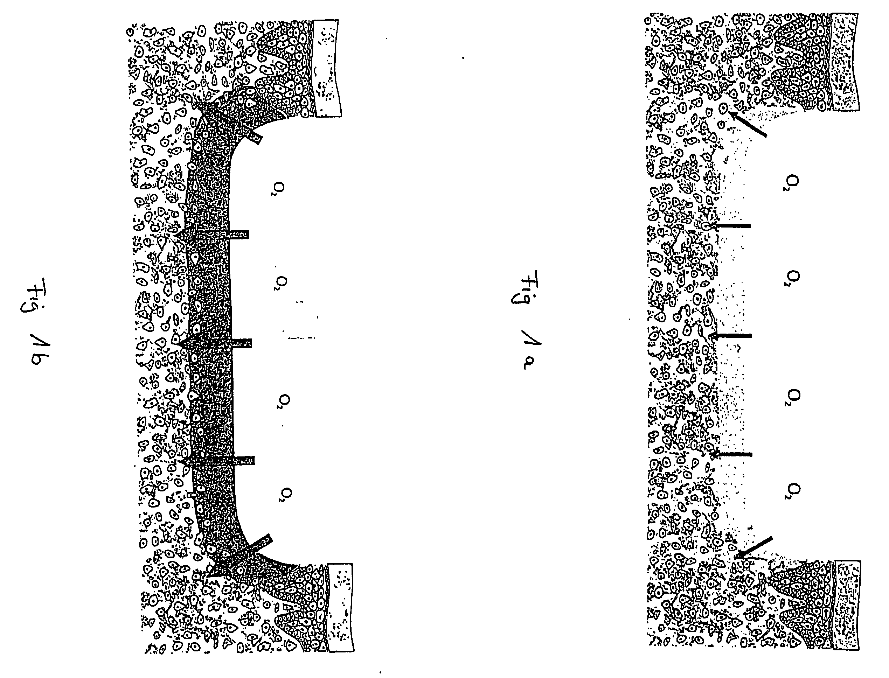

Besteht eine offene Wundfläche, so entfällt die vielseitige diffusive Sauerstoffversorgung der oberflächlichen Zellschicht. Diese ist wie eine Zellkultur. Ihre Sauerstoffversorgung von außen ist deshalb schlecht, weil sich über der Zellschicht ein wässriger Flüssigkeitsfilm bildet, der - wie erklärt - den Gesetzlichkeiten der Diffusion entsprechend, eine diffusive Sauerstoffsperre bildet. Dies veranschaulicht die folgende Figur 1 a, worin die Wasserschicht , die sich über den Zellen des Wundbodens bildet, schematisch angedeutet ist.
Frische Wunden in normalem Gewebe können im günstigsten Fall in einigen Tagen heilen, falls die Sauerstoffversorgung von der Unterseite, also von innen ausreichend ist. Allerdings konnte in tierexperimentellen Studien nachgewiesen werden, dass auch solche frischen Wunden noch etwas besser heilen, wenn man die Sauerstoffkonzentration der umgebenden Luft erhöht (M.P. Pai etal., Sug. Gyn. Obstet. 135 (1972), 756-758). Ältere, insbesondere chronische Wunden lassen sich im Tierversuch nicht simulieren. Beim Menschen heilen sie allerdings bekanntlich mit ihrem ausgeprägten Sauerstoffmangel nur sehr langsam oder überhaupt nicht mehr.

Um nun auch chronische Wunden besser heilen zu können, hat man die sogenannte Hyperbare Sauerstoff-Therapie (HBO) angewandt. Hierbei werden die Patienten in Überdruck-Kammern gebracht, wo sie für eine gewisse Zeit von rund einer Stunde in sogenannten Fahrten einem Überdruck reinen Sauerstoffs von etwa 3 bar ausgesetzt werden. Eine normale Wund-Therapie umfasst ungefähr 40 solcher Fahrten (C.D. Müller et al., Hartmann Wund Forum 1 ('99), 17-25). Tatsächlich werden auf diese Weise Wundheilungen erzielt. Allerdings erweisen sich Mehrfach-Therapien als weniger erfolgreich, und die Wirkung lässt auch mit der Zahl der Fahrten nach. Dies ist erklärlich: Zwar verbessert sich die Sauerstoffversorgung der oberflächlichen Wundzellen, dies wird jedoch - wie oben erläutert- mit einer toxischen Wirkung des konzentrierten hochgespannten Sauerstoffs erkauft; schljeßlich überwiegt vermutlich die schädigende Wirkung.

Das US-Patent 2,527,210 aus dem Jahre 1944 beschreibt eine Hämoglobin - Lösung, welche sowohl intravenös als auch topisch z.B. durch Aufsprühen zur Behandlung von Wunden einsetzbar sein soll. Das Hämoglobin wird hierbei erhalten aus frischen Erythrozyten, welche nach Zentrifugation und Absaugen des überstehenden Blutplasmas eiinem Gefrierschock unterzogen werden. Dadurch erfolgt eine Zelllyse und Hämoglobin wird freigesetzt. Die aufgebrochenen Zellwände liegen im Produkt mit vor. Es handelt sich bei dieser Zubereitung um einen konzentrierten Zell-Detritus (Zelltrümmer). Zur Oberflächenbehandlung soll dadurch insbesondere ein antiseptischer Abdeck - Effekt erzielt werden wie sonst durch Jodlösung, nachdem zuvor noch 5% Natriumsulfid zugesetzt wurde. Hier wird also die Wunde lediglich verschlossen. Um die Viskosität des Produktes z.B. für die Sprühanwendung richtig einzustellen, wird Plasma hinzugefügt. Ein Sauerstofftransport ist hier nicht erwähnt.

Dieser Weg der Anwendung derartiger Hämoglobin- Produkte wurde in der Folgezeit offensichtlich verlassen. So beschreibt die WO 97/15313 den therapeutischen Einsatz von Hämoglobin zur Verbesserung der Wundheilung. Dazu wird Stroma- und pyrogenfreies Hämoglobin systemisch, also intravenös den Patienten insbesondere nach Operationen oder traumatischen Ereignissen verabreicht, um den Blutdruck zu erhöhen. Insbesondere wird ein mit Diaspirin vernetztes Hämoglobin zu diesem Zweck eingesetzt.

Die systemische, intravenöse Verabreichung von Hämoglobin kann jedoch nur den bekannten indirekten einseitigen Einfluss auf die Wundheilung ausüben, da die Versorgung der in der Wunde befindlichen Blutgefäße von innen erfolgen muss und damit von außen keine Möglichkeit der Behandlung und auch keine Überwindung der oben beschriebenen Diffusionssperre möglich ist.
Darüber hinaus ist ein Auftragen von Zellmembranbestandteilen auf offene Wunden fragwürdig, da die aus den Zellmembranen ausströmenden Phospholipide bekanntermaßen teilweise sehr toxisch sind.
In einer am 14.3.2002 im Internet veröffentlichten Mitteilung (www.sangui.de/en/Stock/news: study demonstrates effectiveness of oxygen in skin treatment" wird berichtet, dass mit Emulsionen, enthaltend natürliche oder künstliche Sauerstoffträger, Altershaut oder Falten behandelt werden können.

Allerdings handelt es sich hierbei nicht um offene Wunden, bei denen wie erläutert eine Sperrschicht vorhanden ist.

### Aufgabe der Erfindung

Aufgabe vorliegender Erfindung ist es daher, ein derartiges Produkt zu Behandlung von offenen Wunden einzusetzen, mit welchem Sauerstoff von außen in die betroffenen, insbesondere geschädigten Blutgefäße gezielt lokal transportiert wird, ohne in den gesamten Organismus, also systemisch einzugreifen, wobei unerwünschte Nebenwirkungen vermieden werden können.

### Erläuterung der Erfindung

Diese Aufgabe wird erfindungsgemäß dadurch gelöst, dass ein Mittel bereitgestellt wird, das einen von Plasma und Zellwandbestandteilen befreiten natürlichen oder künstlichen Sauerstoffbinder (oder -träger) oder Mischungen hiervon gemäß Anspruch 1 - letzterer durch eine geeignete Modifikation aus einem natürlichen hergestellt - aufweist und dieses in die wässrige Sauerstoffsperrschicht des Wundbodens eingebracht wird. Hierbei entsteht vermöge der eingebrachten Sauerstoffbinder (-träger) ein effektiver Sauerstofftransport durch die Sperrschicht aufgrund des Mechanismus der erleichterten Diffusion (vgl. Figur1 b).
Überraschenderweise erfolgt hier also nicht ein Abdecken und Verschluss der Wunde. Darüber hinaus ist eine beschleunigte Wundheilung durch die Sauerstoffversorgung der zunächst nicht mehr intakten Blutgefässe von außen möglich. Durch diesen Mechanismus wird den Zellen des Wundbodens der Sauerstoff in physiologischer Weise, nämlich aus der Bindung am Hämoglobin, in nicht toxischer Form und in ausreichender Menge direkt am gewünschten Ort angeboten, eine oxidative Schädigung wird vermieden.
Dies ist vor allem deshalb überraschend, weil der Stand der Technik lehrt, dass eine externe Wundheilung eher mit antiseptischen Mitteln wie Jod oder Hydrogelen (vgl. WO 97/15313, Seite 3, Abs 1) oder aber eine Behandlung von innen heraus erforderlich ist und eine äußere Anwendung von Hämoglobin zum Versiegeln der Wunde führt bzw. eine besonders effektive Wundheilung durch eine äußere Okklusion mit Hypotoxie erreicht werden soll, vgl. Gretenar et al, Schweiz. Med. Forum,2001, 237-242.
Erfindungsgemäß wird ein wie beschrieben natürlicher (nativer) Sauerstoffbinder, ausgewählt aus Hämoglobin oder Myoglobin oder ein modifiziertes Derivat hiervon oder Mischungen hiervon eingesetzt. Die Modifikation kann eine intramolekulare Vernetzung , eine Polymerisation (intermolekulare Vernetzung), eine Pegylierung (kovalente Verknüpfung mit Polyalkylenoxiden), eine Modifikation mit chemisch reaktiven Effektoren wie. Pyridoxal-5'-Phosphat oder 2-Nor-2-Formyl-Pyridoxal-5'-Phosphat, oder auch mit chemisch nicht reaktiven Effektoren der Sauerstoffbindung wie z.B. 2,3-Bisphosphoglycerat, Inositolhexaphosphat, Inositolhexasulfat oder Mellitsäure oder eine Kombination hiervon sein. Für Myoglobin ist keine intramolekulare Vernetzung möglich wie für Hämoglobin, aber alle anderen Modifikationen. Derartige Produkte sind bekannt und beispielsweise in DE-A 100 31 744, DE-A 100 31 742 und DE-A 100 31 740 beschrieben. Vernetzungen von Sauerstoffträgern sind auch in der DE 197 01 37, EP 97 100790, DE 4418 937, DE 38 41 105, DE 37 14 351, DE 35 76 651 beschrieben.
Besonders bevorzugte modifizierte Sauerstoffbinder sind Hämoglobine mit einem Molekulargewicht von 65 000 bis 15 000 000, wie intramolekular vernetzte Produkte wie die gemäß der WO 97115313, insbesondere polymere wie intermolekular vernetzte Produkte mit einem mittleren Molekulargewicht von 80 000 bis 10 000 000 g/Mol, insbesondere 100 000 bis 5 000 000, oder analog hergestellte Myoglobine mit einem Molekulargewicht von 16 000 bis 5 000 000, insbesondere 100 000 bis 3 000 000, bevorzugt bis 1 000 000 g /Mol. Ganz besonders bevorzugt sind solche Sauerstoffträger, die polymerisiert sind zum Beispiel mit für die intermolekulare Umsetzung bekannten Vemetzern wie bifunktionellen Vemetzer wie Butadiendiepoxid, Divinylsulfon, Diisocyanat, insbesondere Hexamethylendiisocyanat, Zyklohexyldiisocyanat und 2,5-Bisisocyanatobenzolsulfonsäure, Di-N-Hydroxysuccinimidylester, Diimidoester, oder Dialdehyd, insbesondere Glyoxal, der analog reagierende Glykolaldehyd, oder Glutardialdehyd.

Femer bevorzugt sind solche Produkte, die auf diese Weise polymersisiert sind und mit einem Polyethylenglykol oder geeigneten Derivat hiervon pegyliert sind. Hierzu gehören beispielsweise Polyethylenoxid, Polypropylenoxid, oder ein Copolymer aus Ethylenoxid und Propylenoxid oder ein Ester, Ether oder Esteramid hiervon. Es ist ferner bevorzugt, wenn das kovalent angeknüpfte Polyalkylenoxid eine Molare Masse von 200 bis 5000 g/mol aufweist.
Zur kovalenten Anknüpfung der Polyalkylenoxide werden bevorzugt solche Derivate der Polyalkylenoxide verwendet, die ein verknüpfendes Agens mit einer funktionellen Gruppe bereits kovalent gebunden enthalten, welche eine direkte chemische Reaktion mit Amino-, Alkohol-, oder Sulfhydryl-Gruppen der Hämoglobine unter Bildung kovalenter Anknüpfungen der Polyalkylenoxide ergeben - beispielsweise Polyalkylenoxide mit reaktiven N-Hydroxysuccinimidylester-, Epoxid- (Glycidylether-), Aldehyd-, Isocyanat-, Vinylsulfon-, Jodazetamid-, Imidazolylformat-, Tresylatgruppen, u. a. Viele solche monofunktionell aktivierte Polyethylenglykole sind kommerziell erhältlich.
Die Herstellung solchermaßen modifizierter Sauerstoffbinder ist in den oben genannten deutschen Patentanmeldungen beschrieben.
Ganz besonders bevorzugt sind modifizierte vernetzte (intra-/ oder intermolekular), oder vemetzte, und pegylierte Hämoglobin - Produkte, mit einem mittleren Molekulargewicht von 250000 bis 750000 g/Mol bzw. Myoglobin - Produkte mit einem mittleren Molekulargewicht von 50000 bis 750000 g/mol. Vor allem sind hierunter solche Produkte bevorzugt, die mit chemisch reaktiven oder mit chemisch nicht reaktiven Effektoren der Sauerstoffbindung oder einer Kombination hiervon zusätzlich umgesetzt sind.
Der Sauerstoffbinder, welcher auch als Sauerstoffträger agiert, kann dabei humanen oder tierischen Ursprungs, wie vom Pferd, Rind oder bevorzugt vom Schwein sein. Dabei wird das Produkt von Plasma und von Zellwandbestandteilen durch geeignete bekannte Maßnahmen wie Zentrifugation und fraktionierte Ultrafiltration befreit gereinigt. Eine Zelllyse durch Tiefgefrieren erfolgt nicht, da andernfalls nicht die gewünschte Zusammensetzung erhalten werden kann. Das Produkt ist darüber hinaus Stroma- und pyrogenfrei.

Insbesondere können die so hergestellten Sauerstoffträger wie beschrieben auch gereinigt werden wie , z. B. chromatographisch (z. B. durch präparative Volumenausschluss-Chromatographie) durch Zentrifugation, Filtration oder Ultrafiltration gereinigt, in Fraktionen unterschiedlichen Molekulargewichts aufgetrennt und nachfolgend weiterverarbeitet werden, vgl. z.B. DE-A 100 31 740 bzw. WO 02/ 00230.
Besonders bevorzugt wird als Sauerstoffbinder humanes oder Schweinehämoglobin, welches natürlich oder wie geschildert modifiziert ist.
Daneben kann auch Myoglobin eingesetzt werden. Bevorzugt ist hierbei natürliches vom Menschen, wobei auch jedes andere tierischer Herkunft oder auch wie geschildert modifiziertes möglich ist. Dieses wird gewonnen wie oben für Hämoglobin beschrieben, wobei jedoch kein intramolar vernetztes möglich ist.
Es können auch Mischungen von natürlichem mit modifiziertem Sauerstoffbinder eingesetzt werden, wie zum Beispiel im Verhältnis 20:1 bis 1:20, bezogen auf das Gewicht.
Auch Mischungen von Myoglobin und Hämoglobin bzw. deren modifizierten Derivate sind möglich, im oben geschilderten Verhältnis von 20:1 bis 1:20.
Das Mittel wird wie nachfolgend näher beschrieben, durch Einbringen des Sauerstoffträgers in ein wässriges Milieu, bereitgestellt.
Erfindungsgemäß erfolgt die Behandlung von offenen, insbesondere chronischen (also auch nicht mehr frischen) Wunden sowohl bei Menschen als auch bei Tieren, wobei die Anwendung beim Menschen bevorzugt ist, durch topische Behandlung mit den beschriebenen Sauerstoffbindem. Zum Auftragen wird der oder die Sauerstoffbinder in einem wässrigen Milieu in einer Menge von 0,1 bis 35 Gew.%, insbesondere 0,1 bis 20, vor allem 0,1 bis 15 Gew.% gelöst. Der Träger, d.h. das wässrige Milieu, vor allem noch physiologisch verträgliche Elektrolyten wie Salze in geeigneten Mengen aufweisen. Hierzu gehören Natriumchlorid, Kalium- Kalzium- Magnesiumchlorid, Natriumhydrogen-(bi)carbonat, Natriumcitrat, Natriumlactat. Dies liegen vorzugsweise in physiologischer Konzentration oder auch Vielfachen hiervon, z. B dem 10fachen, aber auch in Mengen von 0,1 bis 30 Gew.% vor, wobei hierfür insbesondere Natriumchlorid geeignet ist.. Die Elektrolyten können auch im Gemisch vorliegen.

Gegebenenfalls können noch weitere Zusätze, nämlich 0 bis 20, bevorzugt 0,1 bis 20, insbesondere 0 bis 15 Gew.%, bevorzugt 0,1 bis 15, insbesondere 0,1 bis 10 Gew.%, vorliegen. Diese sind insbesondere Nährstoffe für Zellen. Sie werden vor allem ausgewählt aus Glukose, z.B. in Mengen von 0,1 bis 5 Gew.% , Insulin in Mengen von bis zu 25 IE/ml, die für die jeweilige Anwendung bekannten natürlichen Aminosäuren, also die für den Menschen oder für die jeweiligen Tiere bekannten Aminosäuren z.B.0 oder 0,01 bis zu 5 Gew.%, oder auch Gewebefaktoren, wie Interleukine in physiologischen Mengen bis zur 10fachen Menge hiervon.
Gegebenenfalls kann es vorteilhaft sein, wenn als Zusatzstoffe weiterhin Antioxidantien, wie Acetylcystein, Superoxyddismutase in Mengen von 0,001 Gew.% bis 2 Gew.% enthalten sind. In diesem Fall würde, wenn als Sauerstoffbinder Hämoglobin / Derivat eingesetzt wird, dieser auch als Katalase wirken.
Das Mittel wird äußerlich aufgetragen. Je nach Zustand der Wunde wird es eingerieben oder bevorzugt fein aufgesprüht. Dabei kann ein oder mehrere unterschiedliche Sauerstoffbinder eingesetzt werden. So können etwa der oder die natürlichen Sauerstoffbinder in besonders hoher Konzentration, der oder die modifizierten Produkte auch in besonders niedriger Konzentration, je nach Bedarf im Mittel eingesetzt werden oder auch eine Kombination von beiden Gruppen gewählt werden, wenn die Viskosität für den Sprühauftrag besonders eingestellt werden soll. Ansonsten ist die Wahl des oder der Sauerstoffbinder und deren Konzentration jeweils unabhängig und gleichermaßen wirkend.
Erfindungsgemäß hat sich gezeigt, dass offene, insbesondere auch chronische Wunden unterschiedlichster Ursache wirksam behandelt werden können. So kann es sich um solche nach Operationen, nach Traumata, Verletzungen oder auch infolge degenerativer Veränderungen von Gewebe handeln. Hierbei handelt es sich sowohl um degenerative Veränderungen arterieller Blutgefäße als auch Wunden infolge einer chronisch - venösen Insuffizienz. Hierzu gehören insbesondere Dekubitus sowie chronische Wunden, vor allem infolge einer Diabetes - Erkrankung.

### Beispiele

Die Erfindung wird anhand der nachfolgenden Beispiele näher erläutert.

### 1. Herstellung von erfindungsgemäßen Mitteln

### Beispiel 1

Humanes natürliches Hämoglobin wurde durch Zentrifugation und Ultrafiltration vom Plasma und Zellwandbestandteilen befreit und gereinigt.

Hiervon wurden 8 Gew. % in 100 ml Wasser, enthaltend 0,9 Gew. Natriumchlorid sowie 5 Gew. % Glukose und 20 IE/ml Insulin gelöst.

### Beispiel 2

Hochreines Schweinehämoglobin, in einer Konzentration von 330 g/L gelöst in einem wässrigen Elektrolyten der Zusammensetzung 50 mM NaHCO₃ und 100 mM NaCl, wurde bei 4 °C durch Rühren der Lösung unter ständig erneuertem, reinen Stickstoff über der Lösung desoxygeniert. Anschließend wurden 4 mol Natrium-Ascorbat (als 1-molare Lösung in Wasser) pro Mol (monomeren) Hämoglobins zugegeben und 6 h reagieren lassen. Die Lösung wurde mit 0,5-molarer Milchsäure auf einen pH-Wert von 7,1 titriert, 1,1 Mol Pyridoxal-5'-Phosphat je Mol Hämoglobin zugegeben und für 16 h reagieren lassen. Nun wurde mit 0,5-molarer Natronlauge ein pH-Wert von 7,8 eingestellt, 1,1 Mol Natriumborhydrid (als 1-molare Lösung in 0,01-molarer Natronlauge) zugegeben und für eine Stunde reagieren lassen. Jetzt wurde mit 0,5-molarer Milchsäure ein pH von 7,3 eingestellt, zunächst 1,1 Mol 2,3-Bisphosphoglyzerat pro Mol Hämoglobin und nach 15 min Reaktionszeit 8 Mol Glutardialdehyd je Mol Hämoglobin, gelöst in 1,8 L reinem Wasser je Liter Hämoglobinlösung zur Vernetzung des Hämoglobins innerhalb 5 Minuten zugegeben und 2,5 h reagieren lassen. Nach Titration mit 0,5-molarer Natronlauge auf einen pH-Wert von 7,8 folgte eine Zugabe von 15 Mol Natriumborhydrid (als 1-molare Lösung in 0,01-molarer Natronlauge) je Mol Hämoglobin für 1 h. Es erfolgte eine Zugabe von 2 Liter Wasser je Liter ursprünglicher Hämoglobinlösung. Der pH-Wert betrug dann 9,3 und es folgte direkt eine Zugabe von 4 Mol Methoxy-Succinimidylpropionat-Polyethylenglykol des Molekulargewichts 2000 g/Mol für 2 h. Die Stickstoffatmosphäre über der Lösung wurde durch reinen Sauerstoff ersetzt.
Nach 1 h wurden unlösliche Bestandteile durch Zentrifugation (20000 g für 15 min) abgetrennt. Anschließend erfolgte ein Wechsel des Elektrolyten durch eine Volumenausschluss-Chromatographie (Sephadex G-25 - Gel, Pharmacia, D) zu einer wässrigen Elektrolyt-Lösung der Zusammensetzung 125 mM NaCl, 4,5 mM KCI und 20 mM NaHCO₃.
Die Ausbeute betrug 77 %; die Ausbeute für Molekulargewicht größer 700 000 g/Mol ist 28 %.
Messungen der Charakteristik der Sauerstoffbindung unter physiologischen Bedingungen (eine Temperatur von 37 °C, ein Kohlendioxid-Partialdruck von 40 Torr und ein pH-Wert von 7,4) ergaben für das Produkt einen p50-Wert von 22 Torr und einen n50-Wert von 1,95.
Dieser Sauerstoffbinder ist für den erfindungsgemäßen Einsatz in wässriger Lösung wie in Beispiel 1 beschrieben, besonders geeignet.

### Beispiel 3

Die Synthese des mit Glutardialdehyd vemetzten Humanhämoglobins erfolgte wie in Beipiel 2, jedoch unter Verwendung von hochreinem, konzentrierten Humanhämoglobin und Einsatz des 16-fachen molaren Überschusses des Vernetzers. Polymere wurden durch Fraktionieren der Lösung der Vemetzungsprodukte mit Hilfe einer präparativen Volumenausschluss-Chromatographie (gemäß EP-A 95 10 72 80.0: "Verfahren zur Herstellung molekular-einheitlicher hyperpolymerer Hämoglobine" mit Sephacryl S-300 HR - Gel, Pharmacia Biotech, Freiburg, D) gewonnen (hier als die zuerst eluierten 57 Massen-% des vernetzten Hämoglobins).
Die vemetzten Hämoglobine wurden in zwei Teile A und B aufgeteilt. Das Hämoglobin A (vergleiche Abbildung 3) erwies sich als überwiegend polymeres Hämoglobin mit einem Modalwert der Molekulargewichtsverteilung von 950 kg/mol (vergleiche Beispiel 1). Kovalentes Anbinden von monofunktionell aktivem mPEG-SPA-1000 erfolgte analog der in Beispiel 2 für vernetztes Schweinehämoglobin beschriebenen Vorgehensweise. Nach der Addition von Natriumhydrogenkarbonat (bis zu 150 mM) zur Lösung der Polymeren konnte ein 12-facher molarer Überschuss mPEG-SPA-1000 mit den Hämoglobin-Monomeren reagieren. Im Anschluß an eine Reaktionszeit von einer Stunde wurde Lysin im 60-fachen molaren Überschuss zum ,Abfangen' noch aktiver Moleküle des mPEG-SPA-1000 zugegeben. Sowohl das vernetzte Hämoglobin gemäß Lösung A als auch das vernetzte und pegylierte Produkt gemäß Lösung B sind für den erfindungsgemäßen Einsatz geeignet.

### Beispiel 4

Vemetztes Rinderhämoglobin wurde hergestellt durch Vernetzen von hochreinem, konzentriertem Rinderhämoglobin mit einem 14-fachen molaren Überschuss Glutardialdehyd gemäß Beispiel 2, eine molekulare Fraktionierung der Syntheseprodukte, das Anbinden von mPEG-SPA-1000 gemäß Beispiel 2 bzw. 3..
Eine Molekulargewichtsverteilung des nicht modifizierten Hämoglobin-Polymeren zeigt Abbildung 5, nämlich ein Eluogramm einer Volumenausschluss-Chromatographie (am Gel "Sephacryl S-400 HR", Pharmacia Biotech, Freiburg, D), der Modalwert der Molekulargewichtsverteilung beträgt hier 810 kg/mol.

### Beispiel 5

Hochreines, konzentriertes, desoxygeniertes Schweinehämoglobin gelöst in einem wässrigen Elektrolyten der Zusammensetzung 50 mmol/L NaHCO₃ und 100 mmol/L NaCl wurde bei Raumtemperatur mit dem 14-fachen molaren Überschuss an Glutardialdehyd umgesetzt. Natriumcyanoborhydrid, im 10-fachen molaren Überschuss zum (monomeren) Hämoglobin zugesetzt, reduzierte die bei der Vernetzung entstandenen Schiffschen Basen und stabilisierte die kovalente Vernetzung. Die erhaltene Lösung der vemetzten Hämoglobine wurde in drei Teile (A, B und C) geteilt und unterschiedlich weiter verarbeitet.
Teil A blieb unverändert, die Bestimmung der Molekulargewichtsverteilung (gemäß Pötzschke H. et al. (1996, *Macromolecular Chemistry and Physics* 197, 1419 - 1437, sowie Pötzschke H. et al. (1996, *Macromolecular Chemistry and Physics* 197, 3229 - 3250) unter Anwendung der Volumenausschluss-Chromatographie mit dem Gel Sephacryl S-400 HR (Pharmacia Biotech, Freiburg, D) ergab für das vernetzte Schweinehämoglobin einen Modalwert der Molekulargewichtsverteilung von 520 kg/mol.
Die Polymeren des Anteils B wurden mit monofunktionell aktivem mPEG-SPA-1000 (Shearwater Polymers Europe, Enschede, NL) kovalent verknüpft: Zunächst wurde Natriumhydrogencarbonat als Festsubstanz bis zu einer Endkonzentration von 150 mmol/L zur Lösung der vernetzten Hämoglobine addiert, anschließend erfolgte die Zugabe von mPEG-SPA-1000 im 12-fachen molaren Überschuss (bezogen auf die Hämoglobin-Monomeren) ebenfalls als Festsubstanz. Nach einer Reaktionszeit von einer Stunde wurde Lysin im 60-fachem molaren Überschuss (bezogen auf Hämoglobin) zugegeben und reagierte mit noch aktiven mPEG-SPA-1000-Molekülen.
Teil C: Mit der Lösung der vemetzten Hämoglobine wurde genauso verfahren wie für Teil B beschrieben, jedoch unter Verwendung von mPEG-SPA-2000 (Shearwater Polymers Europe, Enschede, NL).
Anschließend erfolgte ein Lösungsmitteltausch in den drei Lösungen A, B und C (mit Hilfe einer Ultrafiltration, "Ultraminisette 10 kDa", Pall Gelman Sciences, Roßdorf, D, oder einer Volumenausschluss-Chromatographie am Gel "Sephadex G-15 M", Pharmacia Biotech, Freiburg, D) zu einer Lösung in einem wässrigen Elektrolyten (StLg) der Zusammensetzung: 125 mM NaCl, 4,5 mM KCI und 3 mM NaN₃.
Alle Produkte gemäß Lösung A, B oder C sind für den erfindungsgemäßen Zweck geeignet.

### Beispiel 6

Intramolekular vernetztes Hämoglobin wurde hergestellt wie in Beispiel 2 beschrieben, jedoch in 0.1 %iger Konzentration.

### Beispiel 7

Käufliches natürliches Humanmyoglobin (z.B. von Sigma,D) wurde gelchromatographisch gereinigt. Dieses kann erfindungsgemäß als solches oder auch modifiziert wie oben beschrieben eingesetzt werden.

### Beispiel 8

10% eines nicht modifizierten Humanhämoglobins wie in Beispiel 1 beschrieben und 5 Gew.% eines wie in Beispiel 2 beschriebenen modifizierten Produktes wurden in 100 ml gereinigtes Wasser, enthaltend 0.9 Gew.% Natriumchlorid, 0,2 Gew.% Natriumbicarbonat, 1 Gew.% Glukose, gegeben. Die Lösung ist sofort gebrauchsfertig.

### Beispiel 9

8 Gew.% eines mit Polyethylenglykol modifizierten Humanmyoglobins, hergestellt gemäß Beispiel 3, Lösung A, wurde in 100 ml gereinigtes Wasser, enthaltend 0,9 Gew.% Natriumchlorid sowie 5 Gew.% Glukose, 20 IE/ml, gegeben.
Die Lösung ist sofort gebrauchsfertig und insbesondere auch haltbar.

### II. Anwendungsbeispiele

### Beispiel 10

Eine Lösung gemäß Beispiel 2 wurde auf eine monatelang bestehende chronische Wunde an der Innenseite der linken Fußfessei einer weiblichen Patientin infolge einer chronisch -venösen Insuffizienz auf die gesamte Fläche dünn mit Hilfe eines Feinsprühers aufgetragen. Dies geschah zweimal täglich. Nach 20 Tagen zeigte sich eine deutliche Granulierung des Wundgrundes unter Konturierung des Wundrandes und Bildung eines zeitweiligen Epithels. Nach 2

Monaten war die Wunde geschlossen.

### Beispiel 11

Bei einem männlichen Patienten bestand 1 Jahr eine 10 cm lange und 4 cm breite Dekiszenz nach arteriellem Verschluß des linken Beines und Absetzen des Vorderfußes (Fischmaul). Es erfolgte eine Behandlung wie in Beispiel 10 beschrieben nach vorheriger Reinigung der chronischen Wunde mit Maden und konzentrierter Harnstofflösung. Nach 4 Monaten war die Wunde verschlossen.

## Patentansprüche

1. Verwendung eines oder mehrerer von Plasma und Zellwandbestandteilen befreiten natürlichen oder modifizierten Sauerstoffbinders, ausgewählt aus Hämoglobin oder Myoglobin menschlichen oder tierischen Ursprungs oder modifizierten Derivaten hiervon, oder Mischungen hiervon zur Herstellung eines Mittels zur externen Behandlung offener Wunden.

2. Verwendung gemäß Anspruch 1, **dadurch gekennzeichnet, dass** der oder die Sauerstoffträger ausgewählt ist aus natürlichem oder modifiziertem humanem oder Schweinehämoglobin oder Mischungen hiervon.

3. Verwendung nach einem der Ansprüche 1 oder 2, **dadurch gekennzeichnet, dass** modifiziertes oder natürliches Myoglobin oder Mischungen hiervon eingesetzt wird.

4. Verwendung nach einem der Ansprüche 1 bis 3, **dadurch gekennzeichnet, dass** Hämoglobin und Myoglobin oder modifizierte Derivate hiervon in einem Mischungsverhältnis von 1:20 bis 20:1 eingesetzt werden.

5. Verwendung gemäß einem der Ansprüche 1 bis 4, **dadurch gekennzeichnet, dass** die Modifikation des oder der Sauerstoffbinder eine intra-, intermolekulare Vernetzung, eine Pegylierung; eine Umsetzung mit chemisch reaktiven oder chemisch nicht reaktiven Effektoren oder eine Kombination hiervon ist.

6. Verwendung gemäß Anspruch 5, **dadurch gekennzeichnet, dass** die Modifikation eine intermolekulare Vernetzung, eine Pegylierung oder eine Kombination hiervon ist.

7. Verwendung gemäß Anspruch 6, **dadurch gekennzeichnet, dass** als Modifikation zusätzlich eine Umsetzung mit einem chemisch nicht reaktiven oder einem chemisch reaktiven Effektor oder einer Kombination hiervon vorliegt.

8. Verwendung gemäß einem der Ansprüche 1 bis 7, **dadurch gekennzeichnet, dass** der oder die Sauerstoffbinder in Form einer Lösung auf die Wunde aufzutragen sind.

9. Verwendung gemäß Anspruch 8, **dadurch gekennzeichnet, dass** der Auftrag des oder der Sauerstoffbinder durch Sprühen zu erfolgen hat.

10. Verwendung gemäß einem der Ansprüche 1 bis 9, **dadurch gekennzeichnet, dass** der oder die Sauerstoffbinder in Form einer wässrigen Lösung, enthaltend physiologisch verträgliche Salze sowie 0,1 bis 15 Gew-% des oder der Sauerstoffbinders und 0 bis 20 Gew.% bzw. in physiologischer bis zur 10-fachen physiologischen Konzentration, Zusatzstoffe, aufzutragen ist.

11. Verwendung gemäß Anspruch 10, **dadurch gekennzeichnet, dass** als physiologisch verträgliche Salze solche, ausgewählt aus Natriumchlorid, Natriumhydrogen- Natriumbicarbonat, Kaliumchlorid, Kalzium-Magnesiumchlorid, Natriumcitrat, Natriumlactat oder Mischungen hiervon enthalten sind.

12. Verwendung gemäß einem der Ansprüche 10 oder 11, **dadurch gekennzeichnet, dass** die Zusatzstoffe ausgewählt sind aus Glucose, Insulin, Aminosäuren, Antioxidantien, Gewebefaktoren

13. Verwendung gemäß einem der Ansprüche 1 bis 12, **dadurch gekennzeichnet, dass** chronische Wunden Operationswunden, Verletzungswunden, Wunden nach Traumata zu behandeln sind.

14. Verwendung gemäß Anspruch 13, **dadurch gekennzeichnet, dass** chronische Wunden infolge einer Degeneration oder Verengung der arteriellen Blutgefäße zu behandeln sind

15. Verwendung gemäß Anspruch 13, **dadurch gekennzeichnet, dass** chronische Wunden infolge einer Diabeteserkrankung zu behandeln sind

16. Verwendung gemäß Anspruch 13, **dadurch gekennzeichnet, dass** DekubitusWunden zu behandeln sind.

17. Verwendung gemäß Anspruch 13, **dadurch gekennzeichnet, dass** Wunden infolge einer chronisch - venösen Insuffizienz zu behandeln sind.

## Claims

1. Use of one or more natural or modified oxygen binders, from which plasma and cell wall constituents have been removed, selected from among haemoglobin or myoglobin of human or animal origin, or modified derivatives thereof, or mixtures thereof for the production of an agent for the external treatment of open wounds.

2. Use according to claim 1, **characterised in that** the oxygen carrier(s) is/are selected from among natural or modified human or porcine haemoglobin or mixtures thereof.

3. Use according to either of claim 1 or claim 2, **characterised in that** modified or natural myoglobin or mixtures thereof is used.

4. Use according to any one of claims 1 to 3, **characterised in that** haemoglobin and myoglobin or modified derivatives thereof are used in a mixture ratio of 1:20 to 20:1.

5. Use according to any one of claims 1 to 4, **characterised in that** modification of the oxygen binder(s) involves intra-/ intermolecular crosslinking, pegylation, reaction with chemically reactive or chemically non-reactive effectors or a combination thereof.

6. Use according to claim 5, **characterised in that** modification involves intermolecular crosslinking, pegylation or a combination thereof.

7. Use according to claim 6, **characterised in that** the modification additionally takes the form of a reaction with a chemically non-reactive or a chemically reactive effector or a combination thereof.

8. Use according to any one of claims 1 to 7, **characterised in that** the oxygen binder(s) is/are to be applied onto the wound in the form of a solution.

9. Use according to claim 8, **characterised in that** the oxygen binder(s) is/are to be applied by spraying.

10. Use according to any one of claims 1 to 9, **characterised in that** the oxygen binder(s) is/are to be applied in the form of an aqueous solution containing physiologically acceptable salts together with 0.1 to 15 wt.% of the oxygen binder(s) and 0 to 20 wt.%, or in physiological concentration up to 10-times physiological concentration, of additives.

11. Use according to claim 10, **characterised in that** the physiologically acceptable salts present are those selected from among sodium chloride, sodium hydrogencarbonate, sodium bicarbonate, potassium chloride, calcium/magnesium chloride, sodium citrate, sodium lactate or mixtures thereof.

12. Use according to either of claim 10 or claim 11, **characterised in that** the additives are selected from among glucose, insulin, amino acids, antioxidants, tissue factors.

13. Use according to any one of claims 1 to 12, **characterised in that** chronic wounds, operation wounds, injury wounds, wounds after trauma are to be treated.

14. Use according to claim 13, **characterised in that** chronic wounds arising from degeneration or narrowing of arterial blood vessels are to be treated.

15. Use according to claim 13, **characterised in that** chronic wounds arising from diabetic disease are to be treated.

16. Use according to claim 13, **characterised in that** decubitus ulcer wounds are to be treated.

17. Use according to claim 13, **characterised in that** wounds arising from chronic venous insufficiency are to be treated.

## Revendications

1. Utilisation d'un ou plusieurs fixateurs d'oxygène naturels ou modifiés débarrassés du plasma et de constituants de la paroi cellulaire, choisis parmi l'hémoglobine ou la myoglobine d'origine humaine ou animale ou de dérivés modifiés de celles-ci, ou de mélanges de celles-ci pour la production d'un agent pour le traitement externe des plaies ouvertes.

2. Utilisation selon la revendication 1 **caractérisée en ce que** le ou les fixateurs d'oxygène sont choisis parmi l'hémoglobine humaine ou de porc naturelle ou modifiée ou les mélanges de celles-ci.

3. Utilisation selon l'une des revendications 1 ou 2 **caractérisée en ce que** de la myoglobine modifiée ou naturelle ou des mélanges de celles-ci sont utilisés.

4. Utilisation selon l'une des revendications 1 à 3 **caractérisée en ce que** l'hémoglobine et la myoglobine ou des dérivés modifiés de celles-ci sont utilisées dans un rapport de mélange de 1:20 à 20:1.

5. Utilisation selon l'une des revendications 1 à 4 **caractérisée en ce que** la modification du ou des fixateurs d'oxygène est une réticulation intra-, intermoléculaire, une pégylation, une réaction avec des effecteurs chimiquement réactifs ou chimiquement non réactifs ou une combinaison de celles-ci.

6. Utilisation selon la revendication 5 **caractérisée en ce que** la modification est une réticulation intermoléculaire, une pégylation ou une combinaison de celles-ci.

7. Utilisation selon la revendication 6 **caractérisée en ce qu'**une réaction avec un effecteur chimiquement non réactif ou un effecteur chimiquement réactif ou une combinaison de ceux-ci existe en outre à titre de modification.

8. Utilisation selon l'une des revendications 1 à 7 **caractérisée en ce que** le ou les fixateurs d'oxygène sont destinés à être appliqués sur les plaies sous forme d'une solution.

9. Utilisation selon la revendication 8 **caractérisée en ce que** l'application du ou des fixateurs d'oxygène est destinée à se produire par pulvérisation.

10. Utilisation selon l'une des revendications 1 à 9 **caractérisée en ce que** le ou les fixateurs d'oxygène sont destinés à être appliqués sous forme d'une solution aqueuse contenant des sels physiologiquement acceptables ainsi que 0,1 à 15 % en masse du ou des fixateurs d'oxygène et 0 à 20 % en masse ou en concentration physiologique jusqu'à 10 fois la concentration physiologique, d'additifs.

11. Utilisation selon la revendication 10 **caractérisée en ce que** sont contenus, comme sels physiologiquement acceptables, ceux choisis parmi le chlorure de sodium, l'hydrogénocarbonate de sodium, le bicarbonate de sodium, le chlorure de potassium, le chlorure de calcium, le chlorure de magnésium, le citrate de sodium, le lactate de sodium ou des mélanges de ceux-ci.

12. Utilisation selon l'une des revendications 10 ou 11 **caractérisée en ce que** les additifs sont choisis parmi le glucose, l'insuline, les aminoacides, les antioxydants, les facteurs tissulaires.

13. Utilisation selon l'une des revendications 1 à 12 **caractérisée en ce que** des plaies chroniques, des plaies opératoires, des plaies de blessure, des plaies consécutives à des traumatismes sont destinées à être traitées.

14. Utilisation selon la revendication 13 **caractérisée en ce que** des plaies chroniques consécutives à une dégénérescence ou un rétrécissement des vaisseaux sanguins artériels sont destinées à être traitées.

15. Utilisation selon la revendication 13 **caractérisée en ce que** des plaies chroniques consécutives à une maladie diabétique sont destinées à être traitées.

16. Utilisation selon la revendication 13 **caractérisée en ce que** des plaies de décubitus sont destinées à être traitées.

17. Utilisation selon la revendication 13 **caractérisée en ce que** des plaies consécutives à une insuffisance veineuse chronique sont destinées à être traitées.
